Europäisches Patentamt

European Patent Office

Office européen des brevets

(19)

(11) Numéro de publication: **0 231 683**
**A1**

(12)

# DEMANDE DE BREVET EUROPEEN

(21) Numéro de dépôt: 86402705.7

(22) Date de dépôt: 05.12.86

(51) Int. Cl.³: **B 01 J 8/06**
//C01B3/38, C07C1/04

(30) Priorité: 06.12.85 FR 8518110

(43) Date de publication de la demande:
12.08.87 Bulletin 87/33

(84) Etats contractants désignés:
AT BE CH DE ES GB GR IT LI LU NL SE

(71) Demandeur: SOCIETE FRANCAISE D'ETUDES
D'INSTALLATIONS SIDERURGIQUES (SOFRESID)
59, rue de la République
F-93104 Montreuil Cédex(FR)

(72) Inventeur: Mazaud, Jean-Paul
51, Avenue du Général Bizot
F-75012 Paris(FR)

(74) Mandataire: Armengaud Ainé, Alain
Cabinet ARMENGAUD AINE 3 Avenue Bugeaud
F-75116 Paris(FR)

(54) **Réacteur échangeur thermique.**

(57) Réacteur-échangeur thermique pour réaliser simultanément un échange thermique entre deux fluides et une réaction chimique par passage du fluide réactant sur un catalyseur, qui comprend une enveloppe délimitant une enceinte dans laquelle sont disposés un faisceau de tubes, une entrée et une sortie pour le fluide de chauffage, et une entrée et une sortie pour le fluide réactant, ce réacteur étant caractérisé en ce qu'il comprend un panier amovible (20) disposé dans ladite enceinte (10) et recevant le catalyseur (22) et le faisceau de tubes (24) au travers duquel circule le fluide de chauffage, ledit panier comportant des parois supérieure et inférieure réalisées sous la forme de plaques tublaires (26, 26') sup-portant le faisceau de tubes, le fluide réactant circulant de haut en bas dans ledit panier, au travers du catalyseur, l'ensemble du panier étant suspendu dans ladite enveloppe et comportant des moyens (55) lui permettant une libre dila-tation.

EP 0 231 683 A1

Réacteur échangeur thermique

La présente invention est relative à un réacteur échangeur thermique conçu de façon à réaliser simultanément un échange thermique entre deux fluides, par exemple entre deux gaz, et une réaction chimique soit exothermique, soit endothermique, par passage du fluide à traiter sur un catalyseur.

On connaît des réacteurs échangeurs répondant à cette préoccupation. Ils comprennent généralement une enveloppe calorifugée délimitant une enceinte dans laquelle est disposé un faisceau de tubes. Dans les réacteurs dont la conception est actuellement connue, les tubes du faisceau sont remplis d'un catalyseur, destiné notamment à provoquer la réaction chimique du fluide à traiter, ou fluide réactant, circulant dans les tubes, et l'on fait circuler au travers de l'enveloppe et autour des tubes un fluide de chauffage.

Il existe aussi des conceptions de réacteurs où le fluide de chauffage circule à l'intérieur des tubes, le catalyseur étant disposé à l'extérieur des tubes.

Les réacteurs thermiques connus présentent un intérêt thermique évident. Par contre, ils présentent des difficultés de mise en oeuvre technologique.

L'invention se propose de réaliser un échangeur thermique dans lequel le catalyseur est disposé à l'extérieur des tubes, et dont les dispositions technologiques permettent d'en faciliter la construction, l'exploitation, l'entretien et la mise en oeuvre du catalyseur.

A cet effet, elle a pour objet un échangeur thermique permettant de réaliser simultanément un échange thermique entre deux fluides, et une réaction chimique par passage du fluide à traiter sur un catalyseur, qui comprend une enveloppe délimitant une enceinte dans laquelle on dispose un faisceau de tubes, une entrée et une sortie pour le fluide de chauffage, et une entrée et une sortie pour le fluide à traiter, ce réacteur étant caractérisé en ce qu'on dispose dans ladite enceinte un panier amovible recevant le catalyseur et le faisceau de tubes au travers desquels circule le fluide de chauffage, ledit panier comportant des parois supérieure et inférieure réalisées sous la forme de plaques tubulaires supportant les faisceaux des tubes, le fluide à traiter ou fluide réactant circulant de haut en bas dans ledit panier, au travers du catalyseur, l'ensemble du panier étant suspendu dans ladite enveloppe et étant muni de moyens lui permettant une libre dilatation.

Selon une caractéristique de cette invention, la tubulure d'admission du fluide réactant est disposée à la partie supérieure de l'enveloppe, et le panier comporte une tuyauterie centrale disposée suivant l'axe du faisceau tubulaire, munie d'ouvertures à sa partie inférieure, au travers desquelles passe le fluide réactant après son passage au travers du catalyseur, de façon que la sortie du fluide réactant traité s'effectue au sommet de l'échangeur, au travers de ladite tuyauterie.

Selon une autre caractéristique de cette invention, les ditalations sont absorbées, d'une part, par des soufflets de dilatation prévus sur le panier, de façon à absorber les dilatations différentielles entre le faisceau de tubes et le panier, et, d'autre part, par la libre suspension du panier, du faisceau tubulaire et de la tuyauterie centrale dans l'enveloppe de l'échangeur, des presse-étoupe ou similaire assurant l'étanchéité.

Selon une autre caractéristique de cette invention, l'ensemble de l'échangeur est conçu démontable, l'enveloppe étant réalisée en deux parties, de manière qu'après démontage des tuyauteries d'amenée et d'évacuation du fluide réactant et de la partie supérieure de ladite enveloppe, on puisse avoir accès à la partie interne de l'échangeur, notamment au panier contenant le catalyseur et les tubes.

3

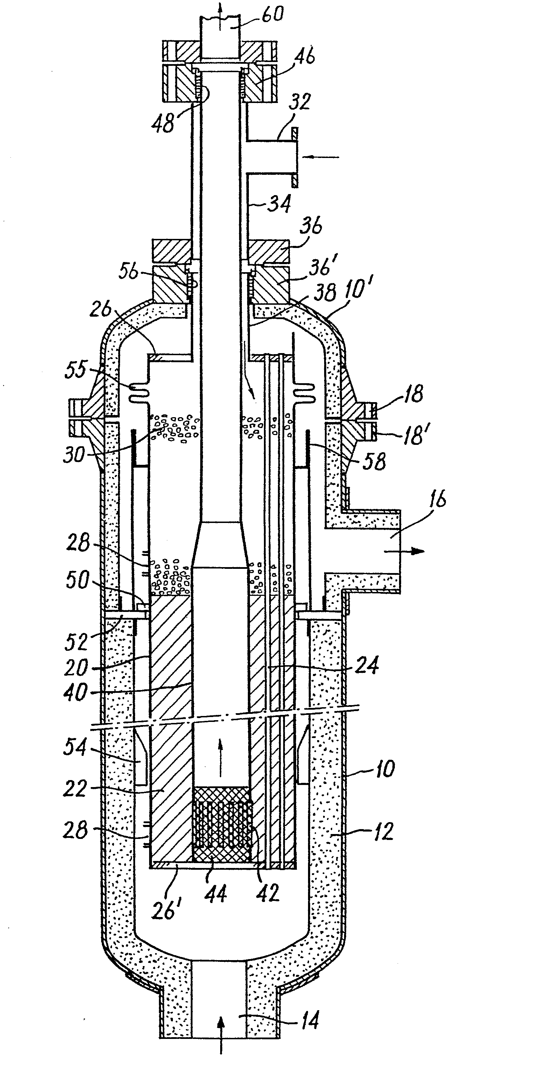

D'autres caractéristiques et avantages de cette invention ressortiront de la description faite ci-après en référence au dessin annexé, dont la figure unique est une vue, en coupe axiale longitudinale, d'un réacteur échangeur selon cette invention.

Le réacteur échangeur comporte une enveloppe 10, constituée par une virole cylindrique verticale, de préférence en acier, munie d'un garnissage réfractaire 12, assurant la résistance à la température et à la pression de l'ensemble de l'échangeur-réacteur. En fonction des applications, ce garnissage peut être réalisé de façons diverses, et il peut être remplacé par exemple par un calorifugeage, notamment lorsque le fluide de chauffage est un liquide. L'enveloppe comporte une entrée 14 et une sortie 16 pour le fluide de chauffage. Comme on peut le voir sur le dessin, l'enveloppe est démontable ; elle comporte en effet deux parties 10, 10', assemblées à l'aide de brides 18, 18'.

Selon cette invention, on dispose, dans l'enceinte délimitée par l'enveloppe 10, un panier 20 amovible contenant, d'une part le catalyseur 22, et, d'autre part, le faisceau tubulaire 24. Ce panier est constitué par une virole cylindrique verticale, munie de plusieurs trappes 28 pour le remplissage et l'extraction du catalyseur, dont les parties inférieure et supérieure sont obturées par des plaques tubulaires 26, 26'. Le panier est réalisé en alliage réfractaire, résistant à la fois aux hautes températures et à l'action chimique du fluide réactant circulant au travers du catalyseur 22.

De préférence, le catalyseur 22 ne remplit pas totalement le volume intérieur du panier 20 ; il est surmonté d'un lit de billes inertes 30, assurant une répartition homogène du fluide réactant sur toute la surface du catalyseur.

Le fluide réactant circule de haut en bas au travers du catalyseur. Il est admis dans l'échangeur par une buse d'entrée 32, munie d'une bride de raccordement débouchant dans une tubulure d'admission 34, montée sur la partie supérieure 10' de l'enveloppe 10 par un système de brides 36, 36'. La tubulure d'admission 34 débouche dans un tube 38, soudé sur la plaque tubulaire 26 et permettant au fluide réactant de pénétrer dans le panier 20. Après son passage au travers du catalyseur, le fluide réactant est repris à la partie inférieure du panier, et renvoyé en tête de l'échangeur. A cet effet, le réacteur comporte une tuyauterie axiale verticale 40, dont la partie inférieure est soudée sur la plaque tubulaire inférieure 26' et est munie d'une pluralité d'ouvertures 42 pour le passage du fluide réactant, ces ouvertures étant protégées par une grille 44 pour retenir le catalyseur 22. A sa partie supérieure, la tuyauterie 40 est munie d'une bride de raccordement 46 à la conduite d'évacuation 60, avec interposition d'un presse-étoupe 48.

L'ensemble de panier 20 est suspendu dans l'enveloppe 10. Il comporte un anneau de support 50, venant reposer sur un anneau interne 52, soudé sur la paroi intérieure de l'enveloppe 10, le centrage du panier dans l'enveloppe étant assuré par des cales, telles que 54, également soudées sur la paroi interne de l'enveloppe, le montage étant tel que le panier et les éléments qu'il supporte puissent se dilater librement.

Selon l'invention, les dilatations sont absorbées par deux moyens :
a) d'une part, par un soufflet de dilatation 55, prévu sur l'enveloppe du panier, afin d'absorber les dilatations différentielles entre les tubes 24 du faisceau et l'enveloppe 20 du panier ;
b) d'autre part, par le montage à libre dilatation des tubes 38 et 40 dans l'enveloppe 10 du réacteur, l'étanchéité étant assurée par la présence des presse-étoupe 48 et 56, ces presse-étoupe n'étant soumis qu'à la différence de pression entre les divers compartiments (perte de charge).

Enfin, dans l'enveloppe 10, on prévoit un joint d'étanchéité à lèvre entre la partie supérieure de l'enveloppe 10 et le panier 20.

L'ensemble du faisceau tubulaire est démontable. Il suffit de démonter les tuyauteries 60,32, et 34, puis la partie supérieure 10' de l'enveloppe 10, pour avoir accès à la partie interne de l'échangeur, qui peut être sortie après destruction de la soudure du joint d'étanchéité 58.

5

Le réacteur-échangeur thermique selon cette invention peut recevoir de nombreuses applications. En effet, il peut s'appliquer à tout échange fluide-fluide, de préférence gaz-gaz, s'accompagnant d'une réaction chimique soit exothermique, soit endothermique : le fluide circulant dans les tubes 24 apportant ou évacuant la chaleur, selon le cas, et le fluide circulant dans le panier 20 pouvant être soit l'effluent, ou une partie de l'effluent, d'un réacteur participant au processus, soit un gaz ou une partie d'un gaz destiné à être traité dans l'appareil par passage au travers du catalyseur, soit un fluide étranger au procédé proprement dit, comme, par exemple, de la vapeur d'eau, soit encore un mélange de gaz de diverses origines.

Le catalyseur peut être tout catalyseur solide convenant à la réaction que l'on cherche à obtenir, quelle que soit la base, quels que soient le ou les éléments actifs.

A titre d'exemples non limitatifs d'applications du réacteur-échangeur selon l'invention, on peut citer :

a) le reformage à la vapeur d'un hydrocarbure gazeux ou vaporisé, utilisant la chaleur portée par l'effluent du reformage secondaire d'une unité de production d'ammoniac, ou la chaleur portée par tout réacteur de reformage auto-thermique, ou la chaleur portée par tout fluide gazeux à haute température ;

b) la méthanisation de gaz de synthèse à faible teneur en oxydes de carbone, par exemple dans une usine d'ammoniac ;

c) la méthanisation de gaz de synthèse à forte teneur en oxydes de carbone, par exemple pour la production de gaz de substitution au gaz naturel ;

d) la combinaison des réactions de conversion et de méthanisation sur un même catalyseur, par exemple pour la production de gaz de substitution au gaz naturel ;

e) la conversion par la vapeur d'eau de gaz porteur de monoxyde de carbone ; et,

f) d'une façon générale, toute réaction catalytique en phase gazeuse, exothermique ou endothermique.

6

On notera que la large gamme d'applications possibles du réacteur-échangeur thermique selon cette invention fait que les températures de fonctionnement ne sont limitées que par la tenue des alliages connus constituant les différentes parties du réacteur. Les presse-étoupe assurant l'étanchéité n'étant soumis qu'à la pression différentielle entre les fluides, la seule limitation concernant les pressions est la différence de pressions qu'autorise le réacteur selon l'invention.

Il demeure bien entendu que cette invention n'est pas limitée à l'exemple de réalisation décrit et représenté, mais qu'elle en englobe toutes les variantes.

1

## Revendications de brevet

1 - Réacteur-échangeur thermique pour réaliser simultanément un échange thermique entre deux fluides et une réaction chimique par passage du fluide réactant sur un catalyseur, qui comprend une enveloppe délimitant une enceinte dans laquelle sont disposés un faisceau de tubes, une entrée et une sortie pour le fluide de chauffage, et une entrée et une sortie pour le fluide réactant, ce réacteur étant caractérisé en ce qu'il comprend un panier amovible (20) disposé dans ladite enceinte (10) et recevant le catalyseur (22) et le faisceau de tubes (24) au travers duquel circule le fluide de chauffage, ledit panier comportant des parois supérieure et inférieure réalisées sous la forme de plaques tubulaires (26, 26') supportant le faisceau de tubes, le fluide réactant circulant de haut en bas dans ledit panier, au travers du catalyseur, l'ensemble du panier étant suspendu dans ladite enveloppe et comportant des moyens (55) lui permettant une libre dilatation.

2 - Réacteur-échangeur selon la revendication 1, caractérisé en ce que la tubulure d'admission (32-34) du fluide réactant est disposée à la partie supérieure de l'enveloppe, et ledit panier amovible (20) comporte une tuyauterie centrale (40) disposée selon l'axe du faisceau tubulaire, munie d'ouvertures (42) à sa partie inférieure, au travers desquelles passe le fluide réactant après son passage au travers du catalyseur, de façon que la sortie du fluide réactant s'effectue, par passage au travers de ladite tuyauterie (40), au sommet de l'échangeur.

3 - Réacteur-échangeur selon l'une des revendiations 1 et 2, caractérisé en ce que les dilatations sont absorbées, d'une part par des soufflets de dilatation (55), prévus de préférence sur le panier, pour absorber les dilatations différentielles entre le faisceau de tubes (24) et l'enveloppe du panier, et, d'autre part, par la libre suspension dudit panier, du faisceau tubulaire et des tubes (38) et (40) dans l'enveloppe de l'échangeur, des presse-étoupe assurant l'étanchéité.

4 - Réacteur-échangeur selon l'une quelconque des revendications précédentes, caractérisé en ce que le panier (20) est supporté par un anneau (50) coopérant avec un anneau de support (52), soudé sur la paroi interne de l'enveloppe (10), et en ce que l'étanchéité entre la partie supérieure et la partie inférieure de l'enceinte de l'enveloppe est assurée par un joint à lèvres (58) soudé.

5 - Réacteur selon l'une quelconque des revendications précédentes, caractérisé en ce que l'enveloppe est réalisée en deux parties séparables (10-10'), de manière qu'après démontage des tuyauteries d'amenée (32-34) et d'évacuation (60) du fluide réactant et de la partie supérieure (10') de l'enveloppe, on puisse avoir accès à la partie interne de l'enveloppe, notamment au panier contenant le catalyseur.

Office européen
des brevets

**RAPPORT DE RECHERCHE EUROPEENNE**

EP  86 40 2705

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| X | US-A-2 276 307  (E.J. HOUDRY) <br> * Page 1, colonne 1, lignes 1-23; page 2, colonne 2, lignes 19-30; page 3, colonne 1, ligne 38 -colonne 2, ligne 75; figure 8 * | 1,4 | B 01 J    8/06 // <br> C 01 B    3/38 <br> C 07 C    1/04 |
| Y |  | 3 |  |
| Y | --- <br> FR-A-2 387 682  (I.C.I.) <br> *  Page 1, lignes 1-2; page 6, lignes 18-34; page 10, ligne 14 - page 11, ligne 8; figure 2 * | 3 |  |
| A | --- <br> FR-A-2 374 076  (DR.C. OTTO & CO.) <br> * Figure 1 * |  |  |
| A | --- <br> EP-A-0 008 633  (LINDE) <br><br> * Résumé; page 17, ligne 13 - page 20, ligne 10; figures 1,5 * |  | **DOMAINES TECHNIQUES RECHERCHES (Int. Cl.4)** <br><br> B 01 J <br> C 01 B <br> C 07 C |

----

Le présent rapport de recherche a été établi pour toutes les revendications

| Lieu de la recherche | Date d'achèvement de la recherche | Examinateur |
|---|---|---|
| LA HAYE | 17-03-1987 | SIEM T.D. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons

& : membre de la même famille, document correspondant

OEB Form 1503 03 82